# EUROPEAN PATENT APPLICATION

(11) **EP 4 545 975 A1**
(43) Date of publication of application: **30.04.2025**
(21) Application number: 24207737.8
(22) Date of filing: 21.10.2024
(51) Int. Cl.: G01N 35/00

(54) **MAGNET PLATE, SEPARATION DEVICE AND AUTOMATIC DISPENSER USING MAGNET PLATE**

(30) Priority: 24.10.2023 JP 2023182829
(71) Applicant: SHIMADZU CORPORATION, Kyoto-shi, Kyoto 604-8511 (JP)
(72) Inventor: MIYAKI, Kosuke, Kyoto-shi, Kyoto, 604-8511 (JP)
(74) Representative: Müller-Boré & Partner Patentanwälte PartG mbB

(57) **Abstract**

A separation device includes a magnet plate on which a well plate having a plurality of wells is placed, which separates a sample utilizing a magnetic force, and which includes a magnet arranged at a side portion of each well to correspond to each well. One magnet is provided at a side portion of one well to correspond to the one well, and a magnetic member is arranged between the one magnet and another well adjacent to the one well.

## Description

### BACKGROUND

### Technical Field

The present disclosure relates to a magnet plate, a separation device, and an automatic dispenser using the magnet plate.

### Description of Related Art

There is a magnet plate that separates a sample contained in a well plate by using a magnetic force of a magnet. Samples contained in a plurality of wells provided in the well plate include magnetic beads. The magnetic beads are collected at a desired position by the magnetic force of the magnet, so that the sample is separated by the magnet plate. In an automatic dispenser using the magnet plate, a dispensing process is executed by suction of a sample separated in a well with use of a tip.

In regard to a magnet plate, there is a type for collecting magnetic beads on the side surface of a well. MagnaStand, which is a magnet plate for 96 well plates, is disclosed at <URL:https://n-genetics.com/products/detail/FG-SSMAG96/> on the Internet (retrieved on September 28, 2023).

### SUMMARY

In regard to the type of a magnet plate that collects magnetic beads on the side surface of a well, in each of all wells, magnetic beads are collected in one location on one side surface of a well. Therefore, there is an advantage that it is easy to perform an operation of sucking a subject to be extracted while avoiding the magnetic beads. In the magnet plate of this type, a plurality of magnets are arranged in a matrix to respectively correspond to a plurality of wells arranged in a matrix. Therefore, a magnet arranged to correspond to a certain well is arranged relatively close to another adjacent well. Therefore, when the positional relationship between the well and the magnet is slightly changed, the other well adjacent to the magnet is also influenced by a magnetic force. Such a state prevents magnetic beads from being collected in one location on one side in the well. Therefore, in regard to the magnet plate of this type, it is necessary to increase the accuracy of arrangement of magnets in order to increase the accuracy of separation of a sample. Alternatively, in order to reduce the influence of magnet on an adjacent well, further contrivance such as using a magnet with a weak magnetic force is required.

An object of the present disclosure is to reduce influence of magnet on an adjacent well in a magnet plate.

A magnet plate according to one aspect of the present disclosure on which a well plate having a plurality of wells is placed and which separates a sample using a magnetic force, includes a magnet that is arranged at a side portion of one or a plurality of wells to correspond to the one or plurality of wells when the well plate is placed. A magnetic member is arranged between the magnet arranged at the side portion of the one or plurality of wells and another well adjacent to the one or plurality of wells.

A separation device according to another aspect of the present disclosure includes a well plate having a plurality of wells, and a magnet plate that separates a sample utilizing a magnetic force. The magnet plate has a magnet arranged at a side portion of one or a plurality of wells to correspond to the one or plu rality of wells, and a magnetic member is arranged between the magnet arranged at the side portion of the one or plurality of wells and another well adjacent to the one or plurality of wells.

The present disclosure is also directed to an automatic dispenser using the above-mentioned magnet plate.

With the present disclosure, it is possible to reduce the influence of magnet on an adjacent well in the magnet plate.

Other features, elements, characteristics, and advantages of the present disclosure will become more apparent from the following description of preferred embodiments of the present disclosure with reference to the attached drawings.

### BRIEF DESCRIPTION OF THE DRAWING

Fig. 1 is a front cross-sectional view of a separation device including a magnet plate according to the present embodiment;
Fig. 2 is a plan view of the separation device according to the present embodiment;
Fig. 3 is a plan view of the separation device, and the structure of the magnet plate located in a lower portion of a well plate is drawn with broken lines in Fig. 3;
Fig. 4 is a plan view of the magnet plate;
Fig. 5 is a perspective view of the magnet plate;
Fig. 6 is a partially enlarged view showing the front cross section of the separation device including the magnet plate;
Fig. 7 is a right side view of a magnet support plate; and
Fig. 8 is a diagram showing a result of experiment in regard to the magnet plate of the present embodiment.

### DETAILED DESCRIPTION

The configuration of a magnet plate 2 and the configuration of a separation device 1 including the magnet plate 2 according to embodiments of the present disclosure will now be described with reference to the drawings.

### (1) Overall Configuration of Separation Device 1

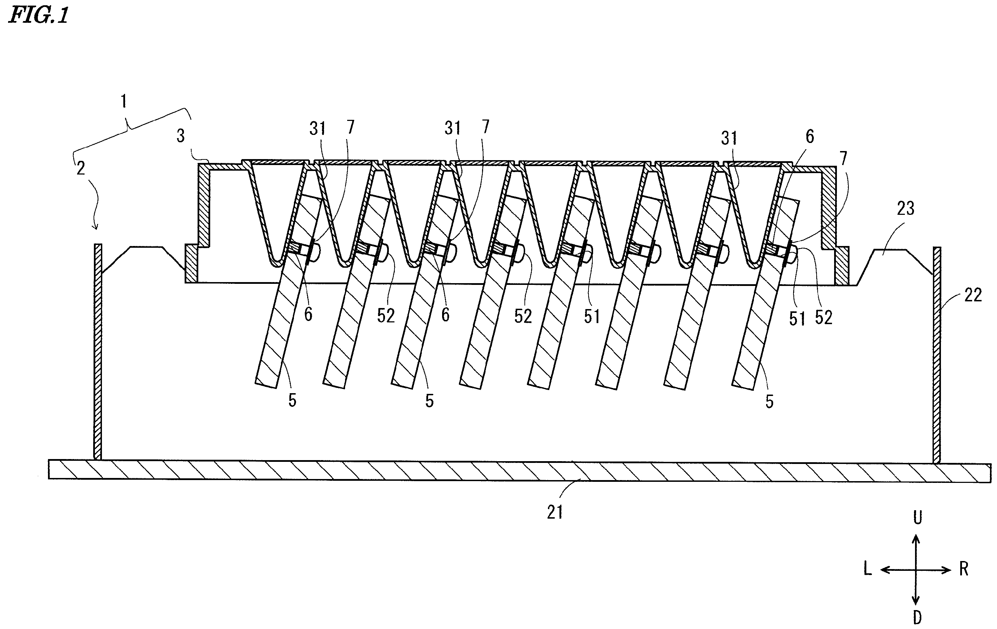

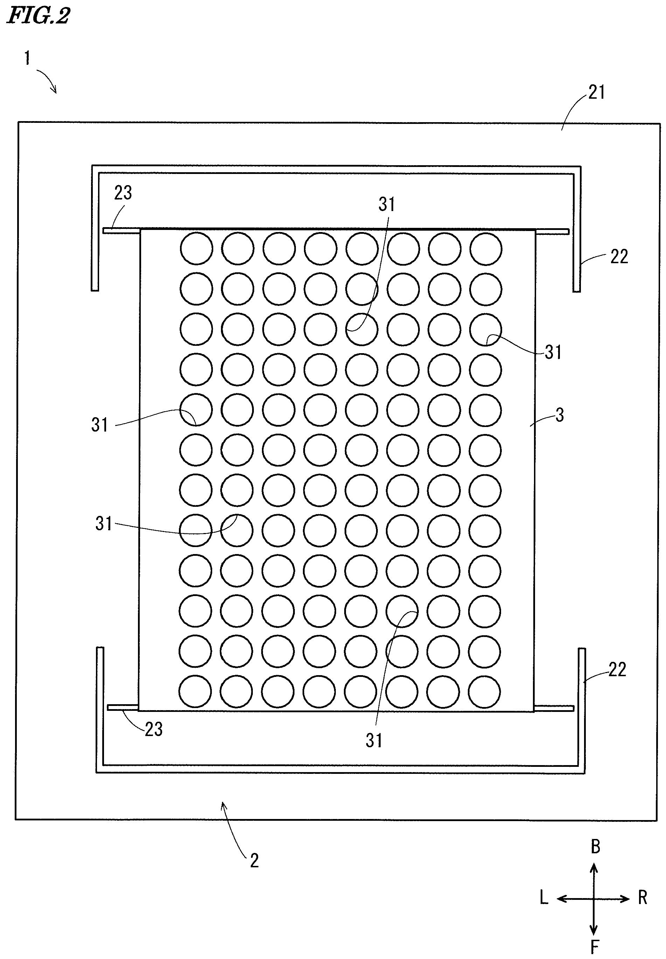

Fig. 1 is a front cross-sectional view showing the separation device 1 according to the present embodiment. The separation device 1 is configured to include the magnet plate 2 and a well plate 3 placed at an upper portion of the magnet plate 2. The separation device 1 including the magnet plate 2 is a device for separating a sample, and is used in an automatic dispenser, for example. The magnet plate 2 separates a sample by attracting magnetic beads included in the sample with utilization of a magnetic force. The particle size, shape, properties and the like of the magnetic beads used in the magnet plate 2 of the present embodiment are not limited in particular. For example, nano-sized fine particles obtained when a magnetic core is coated with a functional group or a biomolecule are utilized as the magnetic beads. In the following description, in Fig. 1, the arrow L indicates a leftward direction, the arrow R indicates a rightward direction, the arrow U indicates an upward direction, and the arrow D indicates a downward direction. The same directions R, L, U and D are also defined in Figs. 2 to 6.

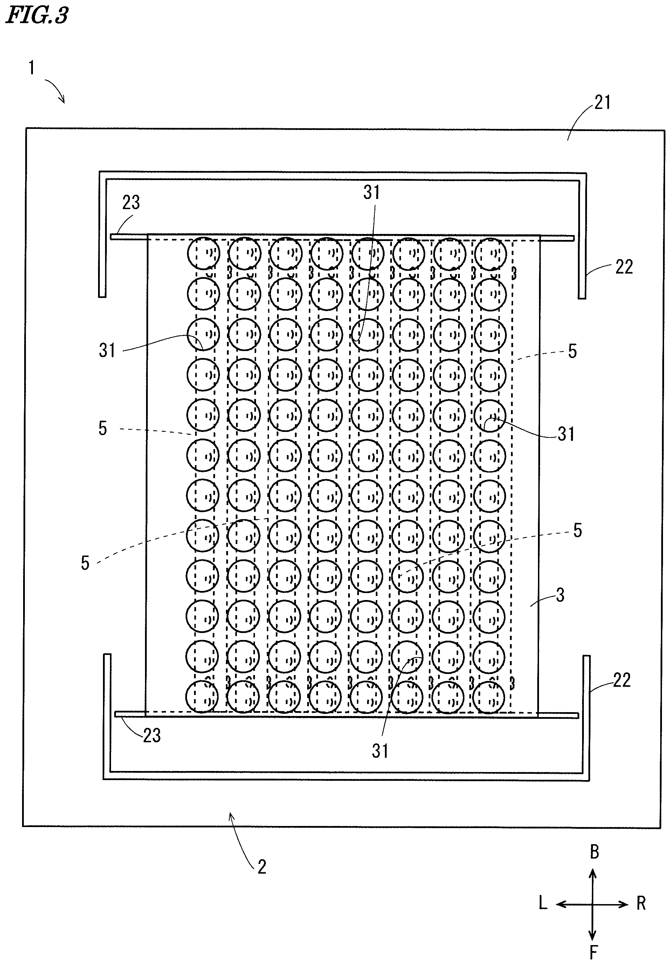

Fig. 2 is a plan view of the separation device 1. As shown in Fig. 2, wells 31 are arranged in a matrix in the well plate 3 included in the separation device 1 of the present embodiment. In the present embodiment, the well plate 3 having 96 wells 31 aligned in 12 rows in a forward-and-rearward direction and 8 columns aligned in a leftward-and-rightward direction is used. In Fig. 2, the arrow F indicates the forward direction, and the arrow B indicates the rearward direction. The similar directions F and B are also defined in the other diagrams of Figs. 3 to 7.

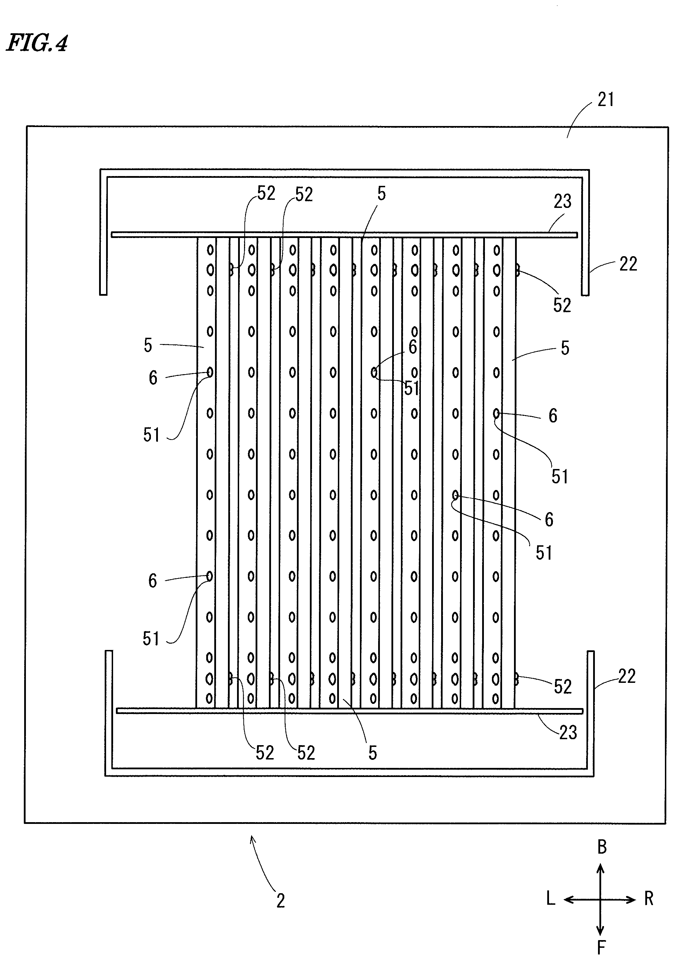

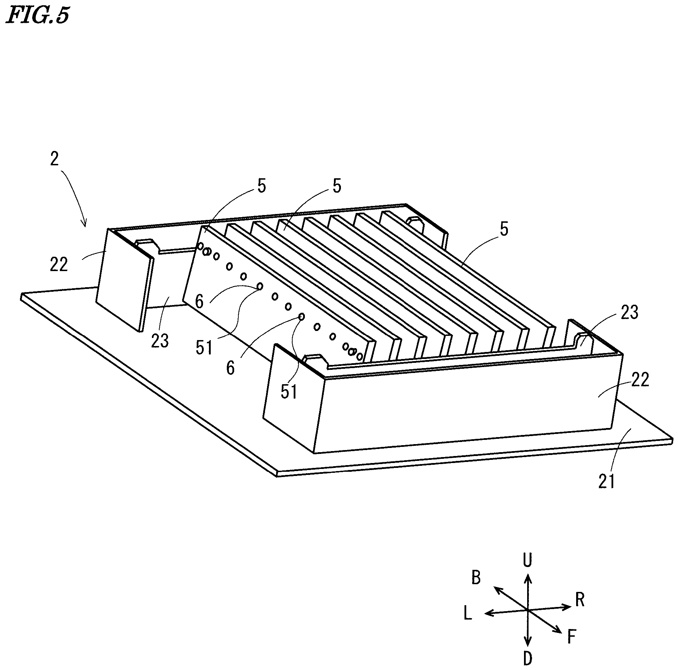

Fig. 3 is a plan view of the separation device 1, and the structure of the magnet plate 2 located in a lower portion of the well plate 3 is drawn with broken lines in Fig. 3. Fig. 4 is a plan view of the magnet plate 2. That is, Fig. 4 is a plan view of the separation device 1 with the well plate 3 detached. Fig. 5 is a perspective view of the magnet plate 2. That is, Fig. 5 is a perspective view of the separation device 1 with the well plate 3 detached. As shown in Figs. 1, and Figs. 3 to 5, 8 magnet support plates 5 are provided in the magnet plate 2. Each magnet support plate 5 is a flat plate-like member extending in the forward-and-rearward direction. For example, an aluminum member can be utilized as the magnet support plate 5. The 8 magnet support plates 5 are arranged at intervals corresponding to the intervals at which 8 wells 31 aligned in the lateral direction of the well plate 3.

As shown in Figs. 4 and 5, the magnet plate 2 has a base 21. Each component of the magnet plate 2 is placed in an upper portion of the base 21. In the base 21, cases 22, 22 for covering the front portion and the rear portion of the magnet plate 2 are provided. Further, in the base 21, support plates 23, 23 for supporting the 8 magnet support plates 5 are provided. The support plates 23, 23 support the front end portion and the rear end portion of each magnet support plate 5. As shown in Figs. 1 and 5, each magnet support plate 5 is supported by the support plates 23, 23 while being inclined with respect to the upward-and-downward direction.

### (2) Support Structure of Magnet

Next, the support structure of the magnet 6 will be described. As shown in Fig. 4, 12 magnet insertion holes 51 are provided in one magnet support plate 5 at equal intervals in the forward-and-rearward direction. Because 8 magnet support plates 5 are arranged in the lateral direction, the total of 12 × 8 = 96 magnet insertion holes 51 are provided in the 8 magnet support plates 5. This 96 magnet insertion holes 51 are provided to correspond to the 96 wells 31 formed in the well plate 3.

As shown in Figs. 1 and 4, the magnet 6 is embedded in each magnet insertion hole 51. That is, 96 magnets 6 are provided to correspond to the 96 magnet insertion holes 51. In the present embodiment, the magnet insertion hole 51 is a cylindrical hole, and the cylindrical magnet 6 is embedded in the magnet insertion hole 51. The magnet 6 is fixed to the left end portion of the magnet insertion hole 51 by an adhesive member, for example.

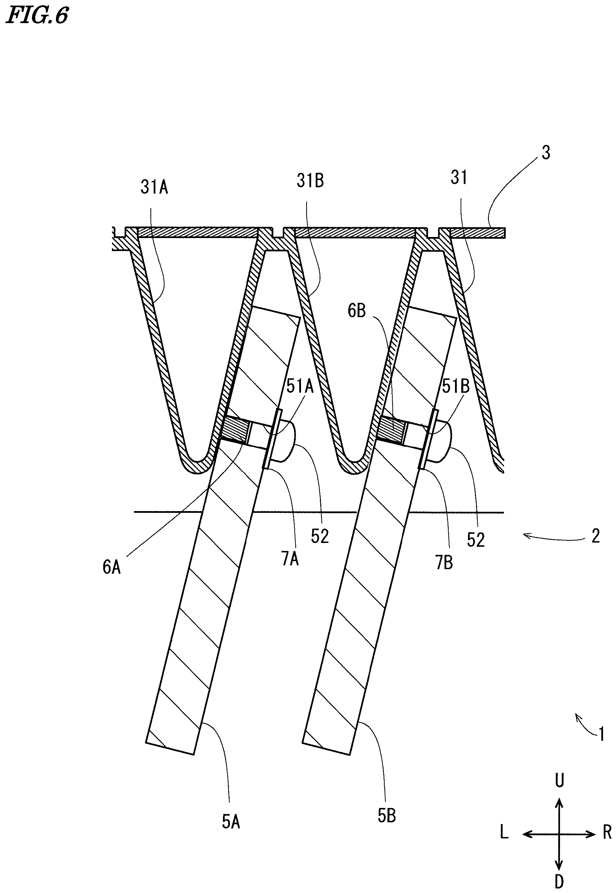

Fig. 6 is a partially enlarged view showing the front cross-section of the magnet plate. That is, Fig. 6 is an enlarged view of the structure of the portion in the vicinity of two magnets 6 in Fig. 1. In Fig. 6, the two wells 31 shown in the diagram are referred to as wells 31A, 31B. Further, the magnet support plates 5 arranged to respectively correspond to the wells 31A, 31B will be respectively described as magnet support plates 5A, 5B. Further, a magnet 6A is embedded in a magnet insertion hole 51A of the magnet support plate 5A, and a magnet 6B is embedded in a magnet insertion hole 51B of the magnet support plate 5B.

As shown in Fig. 6, the magnets 6A, 6B are respectively embedded in the left end portions of the magnet insertion holes 51A, 51B. The left side surface of the magnet support plate 5A is arranged so as to be close to the right side surface of the well 31A. Therefore, the magnet 6A embedded in the left end portion of the magnet insertion hole 51A is close to the right side surface of the well 31A. Similarly, the left side surface of the magnet support plate 5B is arranged so as to be close to the right side surface of the well 31B, and the magnet 6B embedded in the left end portion of the magnet insertion hole 51B is close to the right side surface of the well 31B.

### (3) Support Configuration of Magnetic Plate

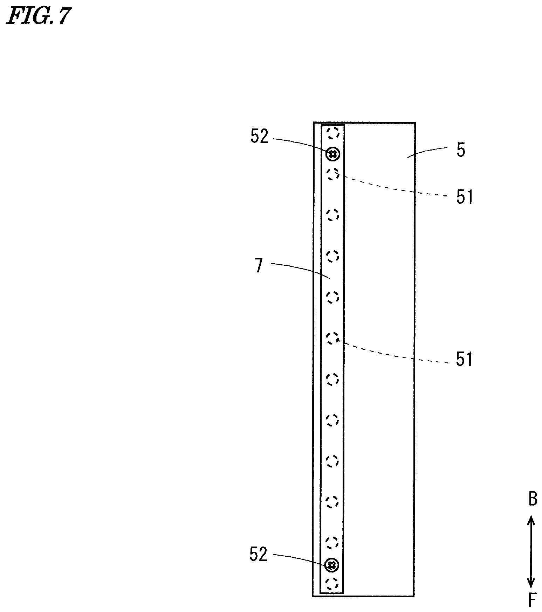

Next, the configuration of a magnetic plate 7 will be described. As shown in Fig. 1, the magnetic plate 7 is provided so as to close the right end portion of the magnet insertion hole 51. Fig. 7 is a right side view of the magnet support plate 5. As shown in Fig. 7, in the magnet support plate 5, the elongated magnetic plate 7 extending in the forward-and-rearward direction is provided. The magnetic plate 7 is provided so as to close the 12 magnet insertion holes 51 arranged in the forward-and-rearward direction. The magnetic plate 7 is attached to the magnet support plate 5 by bolts 52 in two locations arranged in the forward-and-rearward direction.

In the present embodiment, the magnetic plate 7 is made of a stainless steel member. As the magnetic plate 7, stainless steel such as SUS430 can be utilized, for example.

In Fig. 6, the two magnetic plates 7 shown in the diagram are described as magnetic plates 7A, 7B. The magnetic plate 7A is provided so as to close the right end portion of the magnet insertion hole 51A of the magnet support plate 5A, and the magnetic plate 7B is provided so as to close the right end portion of the magnet insertion hole 51B of the magnet support plate 5B.

### (4) Separation of Sample

A method of separating a sample using the separation device 1 that is configured as described above will be described. First, a sample is contained in each well 31 of the well plate 3. As shown in Fig. 6, the magnets 6A, 6B are arranged so as to be close to the right side surfaces of the wells 31A, 31B. Thus, the magnetic beads included in the sample contained in the well 31A are collected on the right side surface of the well 31A by the magnetic force of the magnet 6A. The magnetic beads included in the sample contained in the well 31B are collected on the right side surface of the well 31B by the magnetic force of the magnet 6B. Specifically, the magnetic beads included in the sample are collected on the side surface of each well 31 at a position slightly above the bottom surface of each well 31.

Here, as shown in Fig. 6, in the separation device 1, the well 31A, the magnet 6A, the well 31B and the magnet 6B are arranged in this order from the left. Therefore, the magnet 6B is located adjacent to the right side of the well 31B, and the magnet 6A is located at a small distance from the left side of the well 31B. Therefore, the sample contained in the well 31B may be influenced not only by the magnetic force of the right magnet 6B but also by the magnetic force of the left magnet 6A. However, because the magnetic plate 7A is arranged between the left magnet 6A and the well 31B, the influence of the magnetic force of the left magnet 6A on the sample contained in the well 31B can be reduced. Thus, the sample contained in each well 31 is accurately collected on the right side surface of the well 31 by the magnet 6 arranged adjacent to the right side of the well 31 without being influenced by the magnetic force of the magnet 6 arranged for the adjacent well 31.

With the magnetic beads in each well 31 collected on the right side surface of the well 31 by each magnet 6, the sample is sucked by a tip of the automatic dispenser (not shown) such that the magnetic beads are not sucked. As described above, the magnetic beads are collected on the right side surface of the well 31 at a position slightly above the bottom surface of the well 31. Thus, it is possible to perform a sucking operation while bringing the end of the tip of the automatic dispenser into contact with the bottom surface of the well 31. Thus, a dispensing process can be accurately executed with utilization of the magnet plate 2 of the present embodiment.

There is also a magnet plate of a type in which magnetic beads are collected in a ring shape on the outer peripheral portion of the well 31. When using this type of magnet plate, the automatic dispenser is required to execute the sucking operation while accurately aligning with the center of a ring. In a case in which an amount of sample is small, the diameter of a ring is small. Therefore, it is difficult to perform the sucking operation in particular. With the magnet plate 2 of the present embodiment, magnetic beads are collected in one location on the side surface of the well 31. Therefore, the sucking operation is easily executed by the automatic dispenser. Even in a case in which an amount of sample is small, it is possible to accurately execute the sucking operation using the automatic dispenser with utilization of the magnet plate 2 of the present embodiment.

As described above, in the separation device 1 of the present embodiment, one magnet 6 is provided at the side portion of one well 31 to correspond to the one well 31, and the magnetic plate 7 is arranged between the one magnet 6 and another well 31 adjacent to the one well 31. Thus, it is possible to reduce the influence of the magnetic force of the magnet 6 provided for the other adjacent well 31, and the magnetic beads can be accurately collected on the side surface of the well 31. Thus, because high accuracy is not required for the arrangement of the magnet 6, it is possible to reduce the cost for configuration of the device. Further, a material having a strong magnetic force can be used for the magnet 6, and the configuration for efficiently collecting magnetic beads can be adopted.

### (5) Result of Experiment

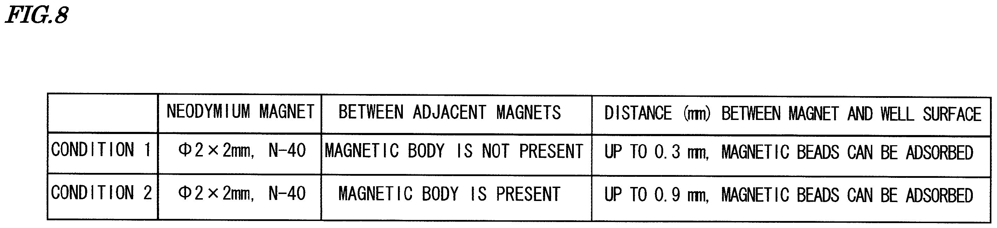

Fig. 8 is a diagram showing the result of experiment using the magnet plate 2 of the present embodiment. In Fig. 8, a condition 1 represents the configuration of a conventional magnet plate in which no magnetic member is arranged between a well and a magnet. A condition 2 represents the configuration of the magnet plate 2 of the present embodiment in which a magnetic member is arranged between a well and a magnet. Further, in Fig. 8, the "DISTANCE BETWEEN MAGNET AND WELL SURFACE" indicates the distance between a magnet and a well surface in a case in which magnetic beads could be accurately collected in one location of the side surface of the well. The "DISTANCE BETWEEN MAGNET AND WELL SURFACE" corresponds to the distance between the magnet 6 and the side surface of the well 31 in the present embodiment. As for the example shown in Fig. 6, the "DISTANCE BETWEEN MAGNET AND WELL SURFACE" indicates the distance between the left end surface of the magnet 6A and the right side surface of the well 31A. Note that neodymium magnets having the same size and the same properties were used for the condition 1 and the condition 2.

As shown in Fig. 8, as for the condition 1 in which no magnetic member was arranged, it was possible to accurately collect magnetic beads by making the "DISTANCE BETWEEN MAGNET AND WELL SURFACE" be close to 0.3 mm. In contrast, as for the condition 2 in which a magnetic member was arranged, even in a case in which the "DISTANCE BETWEEN MAGNET AND SWELL SURFACE" was increased to 0.9 mm, magnetic beads could be accurately collected in one location on the side surface of the well. That is, when a magnetic member is arranged, it is not necessary to increase the accuracy of arrangement of a magnet. With this advantage, it is found that, with the magnet plate 2 of the present embodiment, it is possible to reduce the number of high-precision components as compared with the conventional condition 1, and that the device can be manufactured at lower cost. Further, also in a case in which the shapes of a well plate (PCR plate) and a well are changed, magnetic beads can be stably adsorbed.

Further, with the magnet plate 2 of the present embodiment, a magnet having a magnetic force larger than that of the conventional condition 1 can be used. In a case in which a magnet having a large magnetic force is used, it has the effect of increasing the speed at which the magnetic beads are adsorbed. Alternatively, in a case in which a magnet having a large magnetic force is used, it has the effect that magnetic beads can be adsorbed even when an amount of sample contained in the well plate (PCR plate) is increased.

### (6) Other Embodiments

In the above-mentioned embodiment, the same number of the plurality of magnets 6 are provided to correspond to the plurality of wells 31, by way of example. In the above-mentioned example, the 96 magnets 6 are provided to correspond to the 96 wells 31, by way of example. In another embodiment, one magnet may be provided for a plurality of wells 31. In the above-mentioned embodiment, an elongated magnet extending in the forward-and-rearward direction may be provided to correspond to two or more wells 31 arranged in the forward-and-rearward direction, for example.

In the above-mentioned embodiment, the magnet plate 2 corresponds to the well plate 3 (96-well plate) having the 96 wells 31, by way of example. In addition, the configuration of the magnet plate 2 of the present disclosure can correspond to a well plate having a plurality of wells other than 96 wells.

The magnet plate 2 of the present embodiment can be applied to a PCR well plate that executes a PCR test, for example. By utilizing the magnet plate 2 of the present embodiment, it is possible to improve the dispensing accuracy in the PCR test.

### (7) Correspondences Between Constituent Elements in Claims and Parts in Preferred Embodiments

In the following paragraphs, non-limiting examples of correspondences between various elements recited in the claims below and those described above with respect to various preferred embodiments of the present disclosure are explained. In the above-mentioned embodiment, the leftward-and-rightward direction is an example of a first direction, and the forward-and-rearward direction is an example of a second direction. Further, in the above-mentioned embodiment, the magnet support plate is an example of a support member. Further, in the above-mentioned embodiment, the magnetic plate is an example of a magnetic member.

### (8) Aspects

It will be appreciated by those skilled in the art that the exemplary embodiments described above are illustrative of the following aspects.

(Item 1) A magnet plate according to one aspect of the present disclosure on which a well plate having a plurality of wells is placed and which separates a sample using a magnetic force, includes a magnet that is arranged at a side portion of one or a plurality of wells to correspond to the one or plurality of wells when the well plate is placed, wherein a magnetic member is arranged between the magnet arranged at the side portion of the one or plurality of wells and another well adjacent to the one or plurality of wells.

It is possible to reduce the influence of an adjacent magnet in the magnet plate.

(Item 2) The magnet plate according to item 1, wherein a plurality of magnets corresponding to the plurality of wells may be provided, and the magnetic member may be arranged between one magnet provided at a side portion of one well to correspond to the one well and another well adjacent to the one well.

It is possible to reduce the influence of an adjacent magnet in the magnet plate.

(Item 3) The magnet plate according to item 2, wherein the plurality of wells may be arranged at intervals in a first direction, and the one magnet may be arranged between the one well and the another well in the first direction, and the magnetic member may be arranged between the one magnet and the another well in the first direction.

It is possible to reduce the influence of an adjacent magnet in the magnet plate having the plurality of wells arranged at intervals.

(Item 4) The magnet plate according to item 1, wherein the magnetic member may be attached to a support member supporting the magnet.

A member that supports the magnet can also be used as a member that supports the magnetic member, so that the configuration of the device can be simplified.

(Item 5) The magnet plate according to item 1, wherein the magnetic member may include a stainless member.

It is possible to effectively reduce the influence of an adjacent magnet.

(Item 6) The magnet plate according to item 3, wherein the plurality of wells may be arranged in a matrix at intervals in the first direction and a second direction orthogonal to the first direction, and a plurality of magnets provided to correspond to the plurality of wells arranged in the second direction may be supported by the support member extending in the second direction.

The number of members that support the magnets can be reduced.

(Item 7) The magnet plate according to item 6, wherein the magnetic member may extend in the second direction, and the magnetic member may be arranged between a plurality of magnets arranged in the second direction and a plurality of other wells arranged in the second direction.

The magnetic member can be used to correspond to the plurality of magnets, so that the configuration of the magnet plate can be simplified.

(Item 8) The magnet plate according to item 7, wherein the magnetic member extending in the second direction may be supported by the support member extending in the second direction.

A member that supports the magnet can also be used as a member that supports the magnetic member, so that the configuration of the device can be simplified.

(Item 9) A separation device according to another aspect of the present disclosure includes a well plate having a plurality of wells, and a magnet plate that separates a sample utilizing a magnetic force, wherein the magnet plate has a magnet arranged at a side portion of one or a plurality of wells to correspond to the one or plurality of wells, and a magnetic member is arranged between the magnet arranged at the side portion of the one or plurality of wells and another well adjacent to the one or plurality of wells.

(Item 10) An automatic dispenser according to another aspect of the present disclosure uses the magnet plate according to any one of items 1 to 8.

While preferred embodiments of the present disclosure have been described above, it is to be understood that variations and modifications will be apparent to those skilled in the art without departing the scope of the following claims.

## Claims

1. A magnet plate (2) on which a well plate (3) having a plurality of wells (31) is placed and which separates a sample using a magnetic force, comprising a magnet (6) that is arranged at a side portion of one or a plurality of wells (31) to correspond to the one or plurality of wells (31) when the well plate (3) is placed, wherein
a magnetic member (7) is arranged between the magnet (6) arranged at the side portion of the one or plurality of wells (31) and another well (31) adjacent to the one or plurality of wells (31).

2. The magnet plate (2) according to claim 1, wherein
a plurality of magnets (6) corresponding to the plurality of wells (31) are provided, and
the magnetic member (7) is arranged between one magnet (6) provided at a side portion of one well (31) to correspond to the one well (31) and another well (31) adjacent to the one well (31).

3. The magnet plate (2) according to claim 1 or 2, wherein
the plurality of wells (31) are arranged at intervals in a first direction, and
the one magnet (6) is arranged between the one well (31) and the another well (31) in the first direction, and the magnetic member (7) is arranged between the one magnet (6) and the anotherwell (31) in the first direction.

4. The magnet plate (2) according to any one of claims 1 to 3, wherein
the magnetic member (7) is attached to a support member (5) supporting the magnet (6).

5. The magnet plate (2) according to any one of claims 1 to 3, wherein
the magnetic member (7) includes a stainless member.

6. The magnet plate (2) according to any one of claims 3 to 5, wherein
the plurality of wells (31) are arranged in a matrix at intervals in the first direction and a second direction orthogonal to the first direction, and
a plurality of magnets (6) provided to correspond to the plurality of wells (31) arranged in the second direction are supported by the support member (5) extending in the second direction.

7. The magnet plate (2) according to claim 6, wherein
the magnetic member (7) extends in the second direction, and the magnetic member (7) is arranged between a plurality of magnets (6) arranged in the second direction and a plurality of other wells (31) arranged in the second direction.

8. The magnet plate (2) according to claim 6 or 7, wherein
the magnetic member (7) extending in the second direction is supported by the support member (5) extending in the second direction.

9. A separation device (1) comprising:
a well plate (3) having a plurality of wells (31); and
a magnet plate (2) that separates a sample utilizing a magnetic force, wherein
the magnet plate (2) has
a magnet (6) arranged at a side portion of one or a plurality of wells (31) to correspond to the one or plurality of wells (31), and
a magnetic member (7) is arranged between the magnet (6) arranged at the side portion of the one or plurality of wells (31) and another well (31) adjacent to the one or plurality of wells (31).

10. An automatic dispenser using the magnet plate (2) according to any one of claims 1 to 8.
